# EUROPÄISCHE PATENTSCHRIFT

(11) **EP 0 993 429 B1**
(45) Veröffentlichungstag und Bekanntmachung des Hinweises auf die Patenterteilung: **04.12.2002**
(21) Anmeldenummer: 98933637.5
(22) Anmeldetag: 22.06.1998
(51) Int. Cl.: C07C 37/74, C07C 37/14, C07C 37/52, C07C 39/17

(54) **HERSTELLUNG UND REINIGUNG VON 3-(4-HYDROXYPHENYL)-1,1,3-TRIMETHYLINDAN-5-OL**
METHOD FOR PRODUCING AND PURIFYING 3-(4-HYDROXYPHENYL)-1,1,3-TRIMETHYLINDAN-5-OL
PROCEDE DE PREPARATION ET D'EPURATION DE 3-(4-HYDROXYPHENYL)-1,1,3-TRIMETHYLINDAN-5-OL

(30) Priorität: 03.07.1997 DE 19728378
(43) Veröffentlichungstag der Anmeldung: 19.04.2000
(73) Patentinhaber: BAYER AG, 51368 Leverkusen (DE)
(72) Erfinder: WESTERNACHER, Stefan, Seabrook, TX 77586 (US); LAUKÖTTER, Claudia, D-47877 Willich (DE); STEBANI, Jürgen, D-47800 Krefeld (DE); FENNHOFF, Gerhard, D-47877 Willich (DE)
(86) Internationale Anmeldenummer: EP9803802
(87) Internationale Veröffentlichungsnummer: WO99001413

(56) Entgegenhaltungen:
- US-A- 3 288 864
- US-A- 4 334 106
- US-A- 4 366 328
- DATABASE WPI Section Ch, Week 7532 Derwent Publications Ltd., London, GB; Class E14, AN 75-53040W XP002081238 & JP 50 035150 A (MITSUI TOATSU CHEM INC) , 3. April 1975 in der Anmeldung erwähnt
- DATABASE WPI Section Ch, Week 7930 Derwent Publications Ltd., London, GB; Class A60, AN 79-55597B XP002081239 & JP 54 076564 A (MITSUI TOATSU CHEM INC) , 19. Juni 1979
- DATABASE WPI Section Ch, Week 7830 Derwent Publications Ltd., London, GB; Class A41, AN 78-54215A XP002081240 & JP 53 068762 A (AGENCY OF IND SCI & TECHNOLOGY), 19. Juni 1978

## Beschreibung

Die Erfindung betrifft ein Verfahren zur Herstellung und Reinigung von 3-(4-Hydroxyphenyl)-1,1,3-trimethylindan-5-ol durch Isomerisierung von Isopropenylphenol, dessen Dimeren oder Oligomeren, Abtrennung von 3-(4-Hydroxyphenyl)-1,1,3-trimethylindan-5-ol und anschließende Destillation des Rohprodukts.

Für die Herstellung von 3-(4-Hydroxyphenyl)-1,1,3-trimethylindan-5-ol, im folgenden Bisphenol-Indan genannt, sind bereits verschiedene Verfahren bekannt geworden.

So offenbaren US-A 2 754 285 und US-A 2 819 249 einen Herstellungsweg über die säurekatalysierte Dimerisierung von α-Methylstyrol zu Indanen, welche anschliessend sulfoniert und dann mit Kaliumhydroxid verseift werden.

US-A 2 979 534 lehrt, daß die durch Spaltung von Bisphenolen erhaltenen monomeren Isopropenylphenole in Gegenwart aromatischer Sulfonsäuren oder Mineralsäuren bei Temperaturen von 110 bis 160°C zu Bisphenol-Indan dimerisiert werden können. Bisphenolspaltung und Indanbildung können auch in einem Schritt durchgeführt werden. Nach diesem Verfahren wurde ein Produkt geringer Reinheit erhalten, das auch nach Umkristallisation aus Benzol/Cyclohexan nur einen Schmelzpunkt von 165-166°C aufwies.

US-A 3 264 357 offenbart die Herstellung von Bisphenolen durch Reaktion einer Mischung der beiden isomeren Formen des dimeren Isopropenylphenols mit Phenolen in Gegenwart starker Säuren. Es wird berichtet, daß in Abwesenheit reaktiver Phenole bei einer Reaktionstemperatur von 90°C Bisphenol-Indan gebildet wird. Nach USA 3 264 358 kann Bisphenol-Indan durch Umsetzung einer Mischung der beiden isomeren Formen des dimeren Isopropenylphenols mit stark sauren Katalysatoren erhalten werden, beispielsweise durch zweistündiges Tempern in konzentrierter Salzsäure bei Siedehitze.

US-A 3 288 864 offenbart die Herstellung von Bisphenol-Indan durch Selbstkondensation von monomerem Isopropenylphenol in Gegenwart von Friedel-Crafts-Katalysatoren bei Temperaturen von 50 bis 150°C, JP-A 60/35150 lehrt die Isomerisierung von Isopropenylphenol oder dessen Oligomeren in Gegenwart fester Katalysatoren wie Aluminiumoxid oder Terra alba.

Nach ÜS-A 4 334 106 kann Bisphenol-Indan durch Umsetzung von Isopropenylphenol oder dessen Oligomeren in Halogencarbonsäuren oder Ameisensäure bei Temperaturen von 0 bis 90°C hergestellt werden.

Gemäß JP-A 5/294879 kann Bisphenol-Indan durch thermische Zersetzung von Bisphenol-A in Gegewart von aktiviertem Ton erhalten werden, US-A 3 271 463 offenbart die Bildung von Bisphenol-Indan als Nebenprodukt bei der Behandlung von Bisphenol A mit wäßriger Schwefelsäure bei 90-150°C. Bei beiden Verfahren werden größere Mengen an Spirobisindan-Bisphenol gebildet, welches durch Umkristallisation aus aromatischen Kohlenwasserstoffen abgetrennt werden muß.

Die beschriebenen Verfahren sind für eine industrielle Produktion von Bisphenol-Indan zur Verwendung als Ausgangsstoff für die Kunststoffherstellung vielfach noch unbefriedigend. Um Bisphenol-Indan in der hierfür notwendigen Reinheit zu erhalten, ist eine aufwendige Reinigung durch Umkristallisation erforderlich. Es wurde nun ein Verfahren gefunden, das ohne Umkristallisationsschritt auskommt.

Gegenstand der Erfindung ist ein Verfahren zur Herstellung und Reinigung von Bisphenol-Indan, bei dem man Isopropenylphenol, dessen Dimere oder Oligomere oder deren Gemische in Gegenwart eines sauren Katalysators isomerisiert, aus der Reaktionsmischung rohes Bisphenol-Indan isoliert und dieses Rohprodukt durch Destillation unter vermindertem Druck reinigt, wobei die Destillation in Abwesenheit einer Säure oder Base erfolgt.

Als Ausgangsstoffe für das erfindungsgemäße Verfahren werden Isopropenylphenol oder dessen Dimere oder Oligomere eingesetzt. Diese sind leicht zugänglich und können beispielsweise nach den in US-A 3 288 864 oder US-A 4 201 877 beschriebenen Methoden hergestellt werden.

Die Isomerisierung wird bevorzugt in Gegenwart eines Lösungsmittels durchgeführt. Als Lösungsmittel ist eine Vielzahl unterschiedlicher Lösungsmittel geeignet. Beispiele sind Kohlenwasserstoffe wie Petroläther, Cyclohexan, Benzol, Toluol oder Xylol, Alkohole wie Methanol, n-Propanol oder n-Butanol, Carbonsäuren wie Ameisensäure oder Essigsäure, Propionsäure, Halogencarbonsäuren wie Trichloressigsäure oder Trifluoressigsäure, chlorierte Kohlenwasserstoffe wie Methylenchlorid, Chloroform, Trichlorethylen oder Tetrachlorkohlenstoff, substituierte Aromaten wie Chlorbenzol oder Nitrobenzol; ebenfalls geeignet sind Dimethylsulfoxid, Dimethylformamid, Dimethylacetamid oder N-Methylpyrrolidon. Bevorzugt wird Chlorbenzol verwendet. Die Menge an Lösungsmittel beträgt bevorzugt das zwei- bis dreifache der eingesetzten Menge an Isopropenylphenol.

Die Isomerisierungsreaktion wird in Gegenwart eines sauren Katalysators durchgeführt. Als Katalysatoren für das erfindungsgemäße Verfahren können Brönstedtoder Lewis-Säuren verwendet werden. Beispiele sind Mineralsäuren wie Salzsäure oder Schwefelsäure, organische Säuren wie Sulfonsäuren oder Halogencarbonsäuren, Bortrifluorid und Metallhalogenide wie AlCl₃, FeCl₃ oder ZnCl₂. Daneben können auch heterogene Katalysatorsysteme eingesetzt werden, z.B. in Form eines Festbetts. Beispiele sind saure Ionentauscherharze, Zeolithe, Oxide oder Hydroxide oder Mischoxide von Übergangsmetallen oder seltenen Erden, Heteropolysäuren, Al₂O₃, SiO₂ und deren Mischungen. Bevorzugt werden als Katalysatoren Lewis-Säuren eingesetzt, besonders bevorzugt Bortrifluorid. In einer bevorzugten Ausführungsform des Verfahrens wird der Katalysator der Reaktionsmischung nach Erwärmen auf eine Temperatur im Bereich von 60 bis 110°C, bevorzugt 70 bis 90°C, in einer Menge von 0,002 bis 5 Gew.-%, bevorzugt 0,3 bis 0,5 Gew.-%, bezogen auf die eingesetzte Menge an Isopropenylphenol, gegebenenfalls portionsweise zugesetzt

Die Isomerisierung wird bevorzugt bei einer Temperatur im Bereich von 0 bis 160°C durchgeführt, besonders bevorzugt bei einer Temperatur im Bereich von 110 bis 150°C, ganz besonders bevorzugt bei 130 bis 140°C. Man läßt bevorzugt für 1 bis 600 Minuten, besonders bevorzugt für 2 bis 60 Minuten reagieren.

In einer bevorzugten Ausführungsform wird die Reaktionsmischung im Anschluß an die Isomerisierungsreaktion durch Zusatz einer Base neutralisiert. Die Neutralisation erfolgt bevorzugt bei einer Temperatur im Bereich von 60 bis 100°C, besonders bevorzugt 70 bis 90°C. Zur Neutralisation ist eine Vielzahl verschiedener Basen oder deren Mischungen geeignet. Beispiele sind Metallhydroxide wie NaOH, KOH, Mg(OH)₂, Ca(OH)₂, Alkoholate wie Natriummethanolat, Natriumethanolat, Natriumphenolat, Kaliummethanolat, Kaliumethanolat, Kaliumphenolat, Magnesiumethanolat, Magnesiummethanolat, Magnesiumphenolat, Calciummethanolat, Calciumethanolat, Calciumphenolat, Aluminiumisopropylat, Carboxylate wie Natriumformiat, Natriumacetat, Natriumbenzoat, Calciumformiat, Calciumacetat, Carbonate wie Natriumcarbonat, Kaliumcarbonat, Calciumcarbonat, Magnesiumcarbonat, (NH₄)₂CO₃, Hydrogencarbonate wie NaHCO₃, KHCO₃ oder NH₄HCO₃, Mischungen aus NH₄HCO₃ und Ammomiumcarbamat, Ammoniak, Amine wie Triethylamin, Diethylamin, Ethylamin, Trimethylamin, Dimethylamin, Methylamin und deren Lösungen in Wasser oder organischen Lösungsmitteln, die nicht mit dem Reaktionsmedium mischbar sind. Bevorzugt wird als Base wäßrige Natronlauge verwendet. Wird eine Base verwendet, die mit dem Reaktionsgemisch ein Zweiphasensystem bildet, so wird nach der Neutralisation die das Bisphenol-Indan enthaltende organische Phase abgetrennt.

Aus der Reaktionsmischung wird nach Isomerisierung und gegebenenfalls Neutralisation rohes Bisphenol-Indan isoliert. Vorzugsweise geschieht dies, indem man die Reaktionsmischung mit Wasser versetzt und abkühlt und den sich bildenden Niederschlag von rohem Bisphenol-Indan abtrennt. Die zugesetzte Wassermenge beträgt bevorzugt ein Viertel bis ein Drittel der Menge an Reaktionsmischung. Man kühlt bevorzugt auf eine Temperatur im Bereich von 0 bis 30°C, besonders bevorzugt 0 bis 10°C, ab. Bevorzugt wird die Reaktionsmischung für 1 bis 200 Minuten, besonders bevorzugt 40 bis 80 Minuten bei dieser Temperatur gehalten, bevor man den gebildeten Niederschlag abtrennt. Dies kann durch die dem Fachmann bekannten Methoden geschehen, z.B. durch Filtrieren, Dekantieren oder Zentrifugieren. Vorzugsweise wird der Niederschlag anschließend mit einem organischen Lösungsmittel, z.B. Chlorbenzol, gewaschen.

In einer bevorzugten Ausführungsform des Verfahrens wird der Niederschlag nach der Abtrennung aus der Reaktionsmischung für 5 bis 100 Minuten bei Temperaturen im Bereich von 10 bis 50°C, bevorzugt 20 bis 30°C, in Wasser suspendiert. Die verwendete Wassermenge beträgt dabei bevorzugt das ein- bis zehnfache der Menge an Niederschlag. Anschließend wird der Niederschlag erneut abgetrennt. In einer bevorzugten Ausführungsform wird er dann nochmals bei 10 bis 50°C mit der ein- bis zehnfachen Menge an Wasser gespült. Der erhaltene Feststoff wird getrocknet, bevorzugt bei Temperaturen von 20 bis 150°C, besonders bevorzugt bei 70 bis 90°C. Die Trocknung erfolgt bevorzugt unter vermindertem Druck.

Das Rohprodukt wird anschließend durch Destillation unter vermindertem Druck gereinigt. Obwohl US-A 4 366 328 lehrt, daß sich Bisphenol-Indane schon bei Temperaturen ab 150°C unter Bildung der korrespondierenden Indene zersetzen, wobei die Zersetzungsreaktion unter vermindertem Druck beschleunigt abläuft, gelingt es, aus dem Rohprodukt durch Vakuumdestillation Bisphenol-Indan hoher Reinheit in guter Ausbeute zu erhalten. Bevorzugt wird bei Drücken im Bereich von 10⁻³ bis 10¹ mbar und Temperaturen von 160 bis 230°C destilliert, besonders bevorzugt bei 10⁻² bis 10⁰ mbar und 175 bis 205°C.

Aufgrund seiner hohen Reinheit ist das nach dem erfindungsgemäßen Verfahren hergestellte Bisphenol-Indan in hervorragender Weise geeignet als Ausgangsstoff für die Herstellung von hochwertigen Kunststoffen, z.B. Polycarbonaten.

### Beispiel

800 g dimeres Isopropenylphenol wurden in 2000 ml Chlorbenzol gelöst. Nach Erwärmen der Reaktionsmischung auf 80°C wurden 2,8 ml BF₃ als Etherat zugegeben. Die Reaktionslösung wurde zum Sieden erhitzt und dann für 40 Minuten bei 132°C unter Rückfluß gehalten. Anschließend wurde der Katalysator in der Reaktionslösung durch Zugabe von Natronlauge (1.164 g NaOH auf 300 ml Wasser) bei 80°C neutralisiert. Die wäßrige Phase wurde abgetrennt, die organische Phase auf 0 bis 10°C abgekühlt und mit 600 ml Wasser versetzt. Nach 60 Minuten wurde der Niederschlag abgetrennt und mit 1000 ml Chlorbenzol portionsweise gewaschen. Es wurden 480 g Produkt (entsprechend 60 % der Einwaage an Isopropenylphenol) mit einem Bisphenol-Indan-Gehalt von 89 % erhalten.

Der Rückstand wurde in 2000 ml Wasser suspendiert, nach 15 Minuten wieder isoliert und nochmals portionsweise mit 2000 ml Wasser versetzt. Das Produkt wurde abgetrennt, am Wasserstrahlvakuum bei 80°C getrocknet und anschließend bei einem Druck von 1 mbar destilliert. Bei einer Kopftemperatur von 195 bis 198°C wurden 253 g (entsprechend 53 % der Einwaage an Rohprodukt) Produkt mit einem Bisphenol-Indan-Gehalt von 95 % isoliert.

## Patentansprüche

1. Verfahren zur Reinigung von 3-(4-Hydroxyphenyl)-1,1,3-trimethylindan-5-ol durch Destillation unter vermindertem Druck, wobei die Destillation in Abwesenheit einer Säure und Base erfolgt.

2. Verfahren zur Herstellung und Reinigung von 3-(4-Hydroxyphenyl)-1,1,3-trimethylindan-5-ol, bei dem man
a) Isopropenylphenol, dessen Dimere oder Oligomere oder deren Gemische in Gegenwart eines sauren Katalysators isomerisiert,
b) aus der Reaktionsmischung rohes 3-(4-Hydroxyphenyl)-1,1,3-trimethylindan-5-ol isoliert,
c) das Rohprodukt durch Destillation unter vermindertem Druck, in Abwesenheit einer Säure und Base, reinigt.

## Claims

1. Process for the purification of 3-(4-hydroxyphenyl)-1,1,3-trimethylindan-5-ol by distillation under reduced pressure, the distillation being carried out in the absence of an acid or base.

2. Process for the preparation and purification of 3-(4-hydroxyphenyl)-1,1,3-trimethylindan-5-ol, in which
a) isopropenylphenol, its dimers or oligomers or mixtures thereof is isomerised in the presence of an acid catalyst,
b) crude 3-(4-hydroxyphenyl)-1,1,3-trimethylindan-5-ol is isolated from the reaction mixture,
c) the crude product is purified by distillation under reduced pressure in the absence of an acid or base.

## Revendications

1. Procédé pour purifier le 3-(4-hydroxyphényl)-1,1,3-triméthylindane-5-ol par distillation sous vide, selon lequel la distillation est réalisée en l'absence d'acides et de bases.

2. Procédé pour préparer et purifier le 3-(4-hydroxyphényl)-1,1,3-triméthylindane-5-ol, selon lequel
a) on isomérise l'isopropénylphénol, son dimère ou ses oligomères ou leurs mélanges en présence d'un catalyseur acide,
b) on isole du mélange de réaction le 3-(4-hydroxyphényl)-1,1,3-triméthylindane-5-ol brut,
c) on purifie le produit brut par distillation sous vide en l'absence d'acides et de bases.
